# EUROPÄISCHE PATENTSCHRIFT

(11) **EP 0 543 248 B1**
(45) Veröffentlichungstag und Bekanntmachung des Hinweises auf die Patenterteilung: **13.03.1996**
(21) Anmeldenummer: 92119144.1
(22) Anmeldetag: 09.11.1992
(51) Int. Cl.: C07C 17/12, C07C 25/02

(54) **Verbessertes Verfahren zur Kernchlorierung von aromatischen Kohlenwasserstoffen**
Improved process for the nucleus chlorination of aromatic hydrocarbons
Procédé amélioré pour la chloration dans le noyau d'hydrocarbures aromatiques

(30) Priorität: 21.11.1991 DE 4138243
(43) Veröffentlichungstag der Anmeldung: 26.05.1993
(73) Patentinhaber: BAYER AG, D-51368 Leverkusen (DE)
(72) Erfinder: Schrage, Heinrich, Dr., W-4150 Krefeld 1 (DE); Mais, Franz-Josef, Dr., W-4000 Düsseldorf 1 (DE); Fiege, Helmut, Dr., W-5090 Leverkusen 1 (DE)

(56) Entgegenhaltungen:
- DE-A- 2 536 261
- DE-A- 2 702 829
- US-A- 3 000 975

## Beschreibung

Die Erfindung betrifft ein verbessertes Verfahren zur Kernchlorierung von aromatischen Kohlenwasserstoffen in Gegenwart von Friedel-Crafts-Katalysatoren und in Gegenwart von Co-Katalysatoren in flüssiger Phase.

Die Umsetzung von aromatischen Kohlenwasserstoffen in flüssiger Phase mit gasförmigem Chlor zu kernsubstituierten Chlorderivaten ist bekannt (siehe Ullmanns Enzyklopädie der technischen Chemie, 4. Auflage, Band 9, Seiten 499ff.). Man führt diese Chlorierung im allgemeinen in Gegenwart von Friedel-Crafts-Katalysatoren durch. Als Chlorierungsprodukt wird eine Mischung aus isomeren monochlorierten und polychlorierten Verbindungen erhalten. Bei Verwendung von FeCl₃ und Schwefel als Katalysator bzw. Co-Katalysator erhält man beispielsweise aus Toluol ein Gemisch aus Monochlortoluolen und Dichlortoluolen. In der Monochlortoluolfraktion sind die Hauptprodukte o-Chlortoluol und p-Chlortoluol neben einem geringen Anteil an m-Chlortoluol. Das Verhältnis von o-Chlortoluol zu p-Chlortoluol beträgt etwa 1,1:1.

Da alle Monochlortoluole wertvolle Zwischenprodukte darstellen, wurde auch schon versucht, die Selektivität der Bildung einzelner Isomerer zu erhöhen.

Von besonderer Bedeutung für die Zusammensetzung des Chlorierproduktes ist der verwendete Katalysator, wobei die bekannten Katalysatoren einen großen Spielraum ermöglichen. Eine Übersicht befindet sich in Ullmann's Encyclopedia of Industrial Chemistry, 5th edition, Volume A 6, S. 343.

Häufig wird in technischem Maßstab FeCl₃ als Katalysator eingesetzt, das auf Grund seiner hohen Reaktivität bereits bei niedrigen Konzentrationen einen fast vollständigen Chlorumsatz gewährleistet. Nachteilig ist jedoch, daß relativ viel höher chlorierte Produkte gebildet werden. So enthält das Produkt der Chlorierung von Toluol mit FeCl₃ als Katalysator bei einem Chlorumsatz von 95 Mol-% bereits 9 Gew.-% unerwünschte Dichlortoluole (siehe auch Beispiel 6).

Durch Zusatz von Schwefel läßt sich die Stufenselektivität erhöhen. Gleichzeitig erniedrigt sich aber das Verhältnis von o-Chlortoluol zu p-Chortoluol auf 1,1:1.

Aus der US-PS 3 000 975 ist die Chlorierung von Toluol mit Titantetrachlorid, Zinntetrachlorid, Wolframhexachlorid und Zirkontetrachlorid als Katalysatoren bekannt. Das erreichbare Verhältnis von o-Chlortoluol zu p-Chlortoluol beträgt dabei 3,3:1 und ist damit sehr hoch. Nachteilig bei diesem Verfahren ist jedoch die erforderliche hohe Katalysatorkonzentration von ca. 1 Gew.-% bezogen auf Toluol, die ca. 50mal höher ist als die bei FeCl₃-Katalyse erforderliche Katalysatorkonzentration. Weiterhin ist nachteilig, daß ein hoher Anteil an o-Chlortoluol nur unter völligem Ausschluß von Eisen erreicht wird. Beispielsweise führt ein Zusatz von nur 10 ppm FeCl₃, eine Konzentration, die unter technischen Bedingungen schnell erreicht ist, zu dem gleichen niedrigen Verhältnis von o-Chlortoluol zu p-Chlortoluol und der gleichen schlechten Stufenselektivität wie sie mit reinem FeCl₃ als Katalysator erhalten wird (siehe auch Beispiel 8).

Es besteht also noch immer Bedarf an einem technisch anwendbaren, katalytischen System, mit dem sich gleichzeitig eine gute Stufenselektivität und ein hoher Anteil an o-Chlorverbindungen im Reaktionsgemisch bei geringen Katalysatormengen realisieren läßt.

Es wurde nun ein Verfahren zur Kernchlorierung von aromatischen Kohlenwasserstoffen der Formel (I) 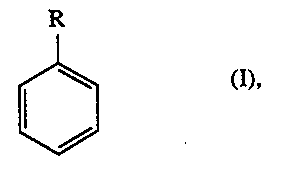
in der
- R: einen C₁-C₁₂-Alkylrest oder C₃-C₈-Cycloalkylrest bedeutet,
in Gegenwart von Friedel-Crafts-Katalysatoren und in Gegenwart von Co-Katalysatoren in flüssiger Phase gefunden, das dadurch gekennzeichnet ist, daß man als Co-Katalysatoren Alkohole einsetzt.

Für die vorliegende Erfindung kommen als Alkohole alle organischen Stoffe in Frage, die eine oder mehrere Hydroxylgruppen enthalten. Beispielsweise seien genannt Alkanole, Phenole, Glykole, partiell veretherte Glykole und Hydroxycarbonsäuren. Aliphatische Hydroxylgruppen enthaltende Verbindungen können beispielsweise 1 bis 24 C-Atome enthalten, aromatische Hydroxylgruppen enthaltende Verbindungen können beispielsweise 6 bis 24 C-Atome enthalten. Bevorzugt sind Alkanole, ganz besonders bevorzugt primäre aliphatische Alkohole mit 1 bis 24 C-Atomen, sowie Gemische derselben. Insbesondere seien genannt: Methanol, Ethanol, Cetylalkohol und Fettalkoholgemische.

In das erfindungsgemäße Verfahren werden vorzugsweise aromatische Kohlenwasserstoffe der Formel (I) eingesetzt, bei denen R für C₁-C₄-Alkyl oder C₅- oder C₆-Cycloalkyl steht. Die Alkylreste können geradkettig oder verzweigt sein. Bevorzugte aromatische Kohlenwasserstoffe der Formel (I) sind: Toluol, Ethylbenzol, Propylbenzol, Cumol, tert.-Butylbenzol und Phenylcyclohexan. Besonders bevorzugt wird in das erfindungsgemäße Verfahren Toluol eingesetzt.

Das erfindungsgemäße Verfahren wird in flüssiger Phase durchgeführt, d.h. der aromatische Kohlenwasserstoff der Formel (I) muß bei Reaktionstemperatur in flüssiger Form vorliegen. Gegebenenfalls kann er zusammen mit einem inerten Lösungsmittel eingesetzt werden. Geeignete Lösungsmittel sind solche, die durch Chlor unter den Bedingungen einer Kernchlorierung nicht angegriffen werden. Beispielsweise kommen chlorierte aliphatische Kohlenwasserstoffe in Frage, wie Methylenchlorid, Chloroform und Tetrachlorkohlenstoff, sowie Essigsäure. Bevorzugt wird jedoch ohne Lösungsmittelzusatz gearbeitet.

Als Chlorierungsmittel für das erfindungsgemäße Verfahren wird vorzugsweise elementares Chlor verwendet. Dieses kann flüssig oder gasförmig in das Reaktionsgemisch eingeleitet werden. Bevorzugt wird gasförmiges Chlor eingesetzt.

Die erfindungsgemäße durchzuführende Kernchlorierung kann grundsätzlich bei beliebigen Temperaturen zwischen dem Erstarrungspunkt und dem Siedepunkt des Reaktionsgemisches durchgeführt werden. Im allgemeinen liegt die Reaktionstemperatur im Bereich zwischen 0 und 100°C, bevorzugt im Bereich zwischen 20 und 80°C, ganz besonders bevorzugt im Bereich von 40 bis 60°C.

Der Druck kann während der Reaktion normal, vermindert oder erhöht sein und ist grundsätzlich unkritisch. Wegen der kostengünstigen Durchführung ist Normaldruck bevorzugt. Erhöhter Druck kann beispielsweise dann angezeigt sein, wenn in Gegenwart eines bei Normaldruck tiefsiedenden Lösungsmittels gearbeitet werden soll. In diesem Fall kann beispielsweise unter dem sich einstellenden Eigendruck des Reaktionsgemisches gearbeitet werden. Die Chlorierung wird bevorzugt so durchgeführt, daß der auf den zu chlorierenden aromatischen Kohlenwasserstoff bezogene Chlorierungsgrad im Reaktionsgemisch 1 nicht übersteigt. Höhere Chlorierungsgrade sind möglich, aber normalerweise nicht vorteilhaft, da sie zur Bildung meist unerwünschter mehrfach chlorierter Produkte führen.

Das Chlorierungsmittel wird daher vorzugsweise in einer Menge von 0,8 bis 1,1, bevorzugt 0,8 bis 1 Mol pro Mol des aromatischen Kohlenwasserstoffs eingesetzt.

Bei den Friedel-Crafts-Katalysatoren handelt es sich vorzugsweise um Eisenverbindungen, insbesondere Eisen(II)-chlorid und Eisen(III)-chlorid. Es kann auch elementares Eisen eingesetzt werden.

Die Mengen des Friedel-Crafts-Katalysators oder eines Gemisches mehrerer von ihnen können in weiten Grenzen variiert werden. So ist bereits bei einem Zusatz von 0,0005 Gew.-% eine katalytische Wirkung erkennbar. Die Obergrenze der Katalysatormenge ist unkritisch, jedoch bieten hohe Mengen im allgemeinen hinsichtlich der Produktzusammensetzung keinen Vorteil, bringen aber häufig Schwierigkeiten bei der Aufarbeitung mit sich. Beispielsweise wird deshalb der Friedel-Crafts-Katalysator in einer Menge von 0,001 bis 0,5 Gew.-%, bevorzugt in einer Menge von 0,01 bis 0,1 Gew.-%, eingesetzt. Alle Mengenangaben sind auf die Menge des eingesetzten aromatischen Kohlenwasserstoffs bezogen.

Das Molverhältnis des Gemisches aus Friedel-Crafts-Katalysator(en) und Alkohol(en) kann im erfindungsgemäßen Verfahren beispielsweise in den Grenzen 1:1 bis 1:20 variiert werden. Im allgemeinen ist es vorteilhaft, Alkohole in einem 2 bis 10fachen molaren Überschuß gegenüber dem Friedel-Crafts-Katalysator einzusetzen. Bevorzugt beträgt das Molverhältnis von Friedel-Crafts-Katalysator zu Alkohol 1:2,5 bis 1:5, besonders bevorzugt 1:2,5 bis 1:3.

Die Reihenfolge der Zugabe der einzelnen Komponenten des Reaktionsgemisches kann beliebig gewählt werden. Das erfindungsgemäße Verfahren kann sowohl kontinuierlich als auch diskontinuierlich durchgeführt werden. Beispielhafte Ausführungsformen des erfindungsgemäßen Verfahrens sind folgende:
a) Ein aromatischer Kohlenwasserstoff der Formel (I), beispielsweise Toluol, wird vorgelegt und auf die gewünschte Temperatur (beispielsweise 50°C) gebracht. Dann gibt man in beliebiger Reihenfolge die gewünschten Mengen an Friedel-Crafts-Katalysator und Alkohol zu und leitet unter weitgehender Konstanthaltung der Temperatur gasförmiges Chlor bis zum gewünschten Chlorierungsgrad ein. Anschließend wird das Gemisch durch Destillation aufgearbeitet.
b) Man stellt eine Mischung aus einem aromatischen Kohlenwasserstoff der Formel (I) mit dem Friedel-Crafts-Katalysator und dem Alkohol her und bringt diese auf die gewünschte Reaktionstemperatur. Dann wird Chlorierungsmittel bis zum gewünschten Chlorierungsgrad zugefügt. Die Aufarbeitung kann auch hier durch Destillation erfolgen.
c) Man stellt eine Lösung von Friedel-Crafts-Katalysator und Alkohol in einem aromatischen Kohlenwasserstoff der Formel (I) her und führt diese einer kontinuierlich arbeitenden Chlorierapparatur zu. Man leitet ebenfalls kontinuierlich das Chlorierungsmittel so schnell ein, daß der gewünschte Chlorierungsgrad erreicht wird. Auch hier kann die kontinuierlich anfallende Reaktionsmischung durch Destillation aufgearbeitet werden.

Das erfindungsgemäße Verfahren gestattet die Herstellung von chlorierten aromatischen Kohlenwasserstoffen mit einer guten Stufenselektivität, einem hohen Anteil von ortho-Chlorverbindungen im Reaktionsgemisch und einem Einsatz von geringen Katalysatormengen. Bei eisenhaltigen Katalysatoren war bisher eine gute Stufenselektivität nur mit Schwefelzusatz zu erzielen, wobei sich gleichzeitig ein niedriger Wert für das Verhältnis von o- zu p-Chlorverbindungen einstellte.

### Beispiele

Prozentangaben sind, soweit nichts anderes gesagt wird, Gewichtsprozent.

### Beispiel 1

In einem geschwärzten Chlorierbecher von 16 cm Höhe und 6 cm Durchmesser mit 4 Wellenbrechern wurden 200 g (2,174 Mol) Toluol bei Raumtemperatur mit 35 mg FeCl₃ und 57 mg n-Pentanol (entspricht einem molaren Verhältnis von 1:3) eingewogen. Danach wurde unter leichtem Stickstoff-Strom auf 50°C erhitzt und mit dem Einleiten von elementarem Chlor direkt unter den Rührer (500 Umdrehungen pro min) begonnen.

Die Chloriergeschwindigkeit betrug etwa 20 Mol-% pro Stunde. Nach 5 Stunden, d.h. nach dem Einleiten von 95 Mol-% Chlor, wurde dem Reaktionsgemisch eine Probe entnommen und gaschromatographisch untersucht. Die Analyse ergab folgende Zusammensetzung:

| | |
|---|---|
| Toluol | 5,3 % |
| o-Chlortoluol | 57,4 % |
| m-Chlortoluol | 0,9 % |
| p-Chlortoluol | 34,9 % |
| Dichlortoluole | 1,4 %. |

### Beispiel 2

Es wurde verfahren wie in Beispiel 1, jedoch wurden statt n-Pentanol 175 mg Octadecanol eingesetzt. Die Produktzusammensetzung nach 5 Stunden war:

| | |
|---|---|
| Toluol | 5,3 % |
| o-Chlortoluol | 57,2 % |
| m-Chlortoluol | 1,1 % |
| p-Chlortoluol | 34,5 % |
| Dichlortoluole | 1,8 %. |

### Beispiel 3

Es wurde verfahren wie in Beispiel 1, jedoch wurden 65 mg n-Propanol (entspricht einem molaren Verhältnis von FeCl₃:n-Propanol von 1:5) eingesetzt. Die Produktzusammensetzung nach 5 Stunden war:

| | |
|---|---|
| Toluol | 5,9 % |
| o-Chlortoluol | 56,2 % |
| m-Chlortoluol | 0,8 % |
| p-Chlortoluol | 34,9 % |
| Dichlortoluole | 1,5 %. |

### Beispiel 4

Es wurde verfahren wie in Beispiel 1, jedoch wurden 130 mg n-Propanol (entspricht einem molaren Verhältnis von FeCl₃:n-Propanol von 1:10) eingesetzt. Die Produktzusammensetzung nach 5 Stunden war:

| | |
|---|---|
| Toluol | 9,4 % |
| o-Chlortoluol | 52,6 % |
| m-Chlortoluol | 0,7 % |
| p-Chlortoluol | 34,0 % |
| Dichlortoluole | 2,0 %. |

### Beispiel 5

Es wurde verfahren wie in Beispiel 1, jedoch wurden statt n-Propanol 169 mg eines technischen Alkoholgemisches (Alfol® 1620, enthaltend C₁₄-C₂₂-Alkohole) eingesetzt. Das Molverhältnis FeCl₃:Alkohol betrug dabei 1:3. Die Produktzusammensetzung nach 5 Stunden war:

| | |
|---|---|
| Toluol | 5,9 % |
| o-Chlortoluol | 56,9 % |
| m-Chlortoluol | 0,8 % |
| p-Chlortoluol | 35,0 % |
| Dichlortoluole | 1,3 %. |

Bei den Beispielen 1 bis 5 lag der Chlordurchbruch stets bei unter 1 %.

### Beispiel 6(zum Vergleich)

Der in Beispiel 1 beschriebene Versuch wurde ohne Zusatz von n-Pentanol durchgeführt. Die Produktzusammensetzung nach 5 Stunden war:

| | |
|---|---|
| Toluol | 10,2 % |
| o-Chlortoluol | 52,8 % |
| m-Chlortoluol | 2,6 % |
| p-Chlortoluol | 25,2 % |
| Dichlortoluole | 9,0 %. |

### Beispiel 7(zum Vergleich)

Es wurde verfahren wie in Beispiel 1, jedoch wurden statt FeCl₃ 100 mg Titantetrachlorid und kein n-Pentanol eingesetzt (siehe US-PS 3 000 975). Die Reaktion verlief wesentlich träger als mit dem FeCl₃-Katalysator. Der Chlordurchbruch betrug 5,3 %. Zusätzlich entstand unerwünschtes Benzylchlorid. Die Produktzusammensetzung nach 5 Stunden war:

| | |
|---|---|
| Toluol | 6,6 % |
| o-Chlortoluol | 65,2 % |
| m-Chlortoluol | 1,2 % |
| p-Chlortoluol | 22,4 % |
| Dichlortoluole | 2,1 % |
| Benzylchlorid | 0,7 %. |

### Beispiel 8 (zum Vergleich)

Es wurde verfahren wie in Beispiel 7 beschrieben und dem Titantetrachlorid 2 mg FeCl₃ zugefügt. Die Produktzusammensetzung nach 5 Stunden war:

| | |
|---|---|
| Toluol | 11,2 % |
| o-Chlortoluol | 55,4 % |
| m-Chlortoluol | 3,6 % |
| p-Chlortoluol | 23,0 % |
| Dichlortoluole | 6,6 %. |

## Patentansprüche

1. Verfahren zur Kernchlorierung von aromatischen Kohlenwasserstoffen der Formel (I) 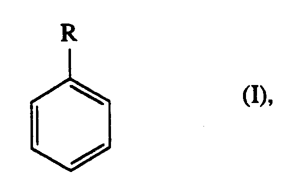 in der
R einen C₁₋C₁₂₋Alkylrest oder C₃₋C₈₋Cycloalkylrest bedeutet,
in Gegenwart von Friedel-Crafts-Katalysatoren und in Gegenwart von Co-Katalysatoren in flüssiger Phase, dadurch gekennzeichnet, daß man als Co-Katalysatoren Alkohole einsetzt.

2. Verfahren nach Anspruch 1, dadurch gekennzeichnet, daß man als Alkohole Alkanole, Phenole, Glykole, partiell veretherte Glykole und/oder Hydroxycarbonsäuren einsetzt.

3. Verfahren nach Ansprüchen 1 und 2, dadurch gekennzeichnet, daß man als aromatische Kohlenwasserstoffe der Formel (I) Toluol, Ethylbenzol, Propylbenzol, Cumol, tert.-Butylbenzol oder Phenylcyclohexan einsetzt.

4. Verfahren nach Ansprüchen 1 bis 3, dadurch gekennzeichnet, daß man als aromatischen Kohlenwasserstoff der Formel (I) Toluol einsetzt.

5. Verfahren nach Ansprüchen 1 bis 4, dadurch gekennzeichnet, daß man die Chlorierung mit elementarem Chlor durchführt.

6. Verfahren nach Ansprüchen 1 bis 5, dadurch gekennzeichnet, daß man eine Reaktionstemperatur im Bereich 0 bis 100°C anwendet.

7. Verfahren nach Ansprüchen 1 bis 6, dadurch gekennzeichnet, daß man als Friedel-Crafts-Katalysatoren Eisenverbindungen einsetzt.

8. Verfahren nach Ansprüchen 1 bis 7, dadurch gekennzeichnet, daß man den Friedel-Crafts-Katalysator in einer Menge von 0,001 bis 0,5 Gew.-%, bezogen auf den eingesetzten aromatischen Kohlenwasserstoff der Formel (I) und den oder die Alkohole im Molverhältnis von 1:1 bis 1:20 (bezogen auf den Friedel-Crafts-Katalysator), einsetzt.

## Claims

1. Process for the ring chlorination of aromatic hydrocarbons of the formula (I) 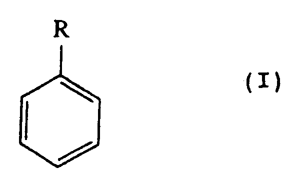 in which
R represents a C₁-C₁₂-alkyl radical or a C₃-C₈-cycloalkyl radical,
in the presence of Friedel-Crafts catalysts and in the presence of co-catalysts in the liquid phase, characterised in that alcohols are used as the co-catalysts.

2. Process according to Claim 1, characterised in that the alcohols used are alkanols, phenols, glycols, partially etherified glycols and/or hydroxycarboxylic acids.

3. Process according to Claims 1 and 2, characterised in that the aromatic hydrocarbons of the formula (I) used are toluene, ethylbenzene, propylbenzene, cumene, tert.-butylbenzene or phenylcyclohexane.

4. Process according to Claims 1 to 3, characterised in that the aromatic hydrocarbon of the formula (I) used is toluene.

5. Process according to Claims 1 to 4, characterised in that the chlorination is performed with elemental chlorine.

6. Process according to Claims 1 to 5, characterised in that a reaction temperature in the range 0 to 100°C is used.

7. Process according to Claims 1 to 6, characterised in that the Friedel-Crafts catalysts used are iron compounds.

8. Process according to Claims 1 to 7, characterized in that the Friedel-Crafts catalyst is used in an amount of 0.001 to 0.5% by weight, relative to the aromatic hydrocarbon of the formula (I) which is used, and the alcohol or alcohols is/are used in a molar ratio of 1:1 to 1:20 (relative to the Friedel-Crafts catalyst).

## Revendications

1. Procédé pour chlorer dans le noyau des hydrocarbures aromatiques de formule (I) : 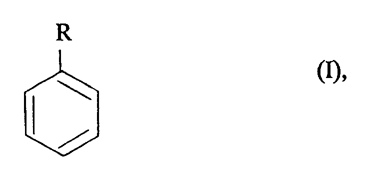 dans laquelle
R représente un groupe alkyle en C₁ à C₁₂ ou cycloalkyle en C₃-C₈,
en présence de catalyseurs de Friedel-Crafts et en présence de catalyseurs auxiliaires, en phase liquide, caractérisé en ce que l'on utilise des alcools en tant que catalyseurs auxiliaires.

2. Procédé selon la revendication 1, caractérisé en ce que les alcools utilisés consistent en alcanols, phénols, glycols, glycols partiellement éthérifiés et/ou acides hydroxycarboxyliques.

3. Procédé selon les revendications 1 et 2, caractérisé en ce que l'on met en oeuvre en tant qu'hydrocarbures aromatiques de formule (I) le toluène, l'éthylbenzène, le propylbenzène, le cumène, le tert-butylbenzène ou le phényl-cyclohexane.

4. Procédé selon les revendications 1 à 3, caractérisé en ce que l'hydrocarbure aromatique de formule (I) mis en oeuvre est le toluène.

5. Procédé selon les revendications 1 à 4, caractérisé en ce que l'on chlore à l'aide du chlore élémentaire.

6. Procédé selon les revendications 1 à 5, caractérisé en ce que l'on observe une température de réaction dans l'intervalle de 0 à 100°C.

7. Procédé selon les revendications 1 à 6, caractérisé en ce que les catalyseurs de Friedel-Crafts mis en oeuvre sont des composés du fer.

8. Procédé selon les revendications 1 à 7, caractérisé en ce que l'on met en oeuvre le catalyseur de Friedel-Crafts en quantité de 0,001 à 0,5 % en poids, par rapport à l'hydrocarbure aromatique de formule (I) mis en oeuvre, et le ou les alcools à un rapport molaire de 1:1 à 1:20 (par rapport au catalyseur de Friedel-Crafts).
